# EUROPÄISCHE PATENTSCHRIFT

(11) **EP 0 530 447 B1**
(45) Veröffentlichungstag und Bekanntmachung des Hinweises auf die Patenterteilung: **22.10.1997**
(21) Anmeldenummer: 92108912.4
(22) Anmeldetag: 27.05.1992
(51) Int. Cl.: C07K 1/16, C12P 21/08, A61K 39/395, G01N 33/577

(54) **Verfahren zur Reinigung von IgG-monoklonalen Antikörpern**
Process for purifying IgG monoclonal antibodies
Procédé de purification d'anticorps monoclonaux du type IgG

(30) Priorität: 08.06.1991 DE 4118912
(43) Veröffentlichungstag der Anmeldung: 10.03.1993
(73) Patentinhaber: BIOTEST PHARMA GMBH, D-63303 Dreieich (DE)
(72) Erfinder: Kothe, Norbert, Dr., W-6242 Kronberg 1 (DE); Rudnick, Dieter, Dr., W-6072 Dreieich (DE); Rohm, Detlef, Dr., W-64367 Mühltal (DE); Bethke, Ulf, Dr., W-6074 Rödermark (DE); Kloft, Michael, Dr., W-6100 Darmstadt (DE)
(74) Vertreter: Beil, Hans Chr., Dr.

(56) Entgegenhaltungen:
- EP-A- 0 268 973
- EP-A- 0 270 025
- EP-A- 0 377 855
- WO-A-86/04486
- WO-A-89/05157
- BIOCHROMATOGRAPHY, Bd. 4, Nr. 1, 1989, NATICK,MASS.,USA, Seiten 4 - 18; NAU: 'CHROMATOGRAPHIC METHODS FOR ANTIBODY PURIFICATION AND ANALSYSIS'

## Beschreibung

Die vorliegende Erfindung betrifft das in den Ansprüchen beschriebene Verfahren zur Reinigung von IgG-monoklonalen Antikörpern sowie deren Verwendung, insbesondere deren intravenöse Anwendung in der Immuntherapie.

Monoklonale Antikörper werden von Hybridomzellen produziert und zunehmend in den Bereichen Diagnostik und Analytik sowie als Therapeutikum eingesetzt.

Die die Antikörper produzierenden Hybridomzellen werden üblicherweise in vivo als Ascites-Tumor oder in vitro in Nährmedium vermehrt.

Die auf diese Weise erhaltenen Antikörper-haltigen Lösungen sind mit Bestandteilen der Ascitesflüssigkeiten und der Kulturmedien kontaminiert.

Speziell für den therapeutischen Einsatz ist eine hohe Reinheit der monoklonalen Antikörper gefordert, so daß dem Reinigungsverfahren eine entscheidende Bedeutung zukommt.

Bei der in vivo Vermehrung von Hybridomzellen ist die Menge der zu gewinnenden Ascitesflüssigkeit limitiert, so daß nur begrenzte Mengen an monoklonalen Antikörpern gewonnen werden können. Ferner sind die monoklonalen Antikörper vor allem mit Proteinen und möglicherweise mit Viren des Wirtsorganismus verunreinigt, was intensive Reinigungsverfahren erfordert.

Nahezu beliebige Mengen von monoklonalen Antikörpern lassen sich gewinnen, wenn die Vermehrung der Hybridomzellen in vitro in Kulturmedien vorgenommen wird. Üblicherweise enthalten die Kulturmedien heterologe Seren oder Fraktionen davon als Wachstumsfaktor, die beim Aufreinigungsprozeß vollständig wieder entfernt werden müssen.

Für die Anwendung von monoklonalen Antikörpern als Therapeutikum bestehen besondere Anforderungen bezüglich Reinheit, DNA-Gehalt, Virussicherheit und biologischer Funktion.

Das Reinigungsverfahren für die Antikörperpräparation muß demnach gewährleisten, daß der DNA-Gehalt kleiner als 10 pg pro Dosis, die Reinheit größer als 99% ist, das Verfahren möglicherweise vorhandene Viren um mindestens 12 log 10 reduziert und die biologische Funktion erhalten bleibt.

Chromatographische Methoden zur Reinigung von monoklonalen IgG-Antikörpern sind in der Literatur beschrieben.

Eine Übersicht über die derzeit angewandten Methoden ist in Commercial Production of Monoclonal Antibodies, edited by Sally S. Seaver, Marcel Dekker, Inc., New York, Basel, 1987, gegeben.

In obengenannter Druckschrift und in der EP 0 310 719 wird ein Reinigungsverfahren für monoklonales IgG mit Affinitätsmedien auf der Basis von Protein-A beschrieben. Protein-A-Säulen weisen jedoch einige Nachteile auf, die insbesondere für den Einsatz zur Reinigung von therapeutisch einsetzbaren monoklonalen Antikörpern ungeeignet sind. Das als Affinitätsgruppe eingesetzte bakterielle Staphylokokken-Protein-A wird beim Betrieb der Säule losgelöst und kontaminiert die zu infundierende monoklonale Antikörper-Lösung.

Dies ist insofern problematisch, da die zu reinigenden Kulturüberstände mit proteolytischen Enzymen verunreinigt sind, die diese Liganden spalten oder vom Träger loslösen.

Die Elution des gebundenen monoklonalen Antikörpers von der Affinitätssäule erfolgt bei niedrigen pH-Werten. Unter diesen Bedingungen besteht die Gefahr, daß insbesondere labile monoklonale Antikörper ihre Struktur verändern, sogar denaturieren und damit ihre biologische Funktion verlieren.

Da es ferner erforderlich ist, daß alle für die Reinigung eingesetzten Geräte und Materialien, unter anderem die Chromatographiephasen, sterilisierbar sind, damit die MAK-haltigen Lösungen steril und pyrogenfrei sind, wenn sie intravenös angewendet werden sollen, besteht die Gefahr, daß eine solche Stabilität nicht gegeben ist, da Protein-A unter den erforderlichen Sterilisationsbedingungen selbst denaturiert.

In der EP-A 0 144 028 und der US-PS 4 746 183 sind ebenfalls chromatographische Methoden mit einem spezifisch modifizierten Kieselgel bzw. unter Verwendung von Hydroxylapatit sowie Protein A zur Reinigung von MAK beschrieben. Die hierbei erzielte Reinheit (etwa 90%, vgl. EP-A 0 144 028) reicht jedoch nicht aus, um die Qualitätsanforderungen an MAK's für einen therapeutischen, insbesondere intravenösen Einsatz zu erfüllen. Ferner ist das gewählte Chromatographie-Material sehr empfindlich und teuer.

In "Biochromatography" (1989), S. 4-18 ist eine Übersicht über Verfahren zur Reinigung von monoklonalen Antikörpern mittels chromatographischer Verfahren aufgezeigt, wobei insbesondere auf die Schwierigkeiten einer Anionen- und Kationenaustauschchromatographie hingewiesen wird.

Die WO 89/05157 beschreibt ein Verfahren zur Reinigung monoklonaler Antikörper mittels Kationen- und Anionenaustauschschritt, wobei jedoch keine Reinheitskriterien angegeben sind.

Der vorliegenden Erfindung liegt daher die Aufgabe zugrunde, ein Reinigungsverfahren für IgG-monoklonale Antikörper bereitzustellen, durch das zuverlässig und wirtschaftlich eine hochreine Antikörper-Lösung erhalten wird, die insbesondere für die intravenöse Anwendung in der Immuntherapie geeignet ist.

Diese Aufgabe wird erfindungsgemäß dadurch gelöst, daß man drei Chromatographieschritte - Anionaustausch, Kationenaustausch und hydrophobe Phase - mit einem spezifischen Sterilisationsschritt unter den jeweils geeigneten Bedingungen kombiniert. Dabei wird - unter Einsatz billiger und leicht verfügbarer Materialien - eine Antikörper-Lösung mit einer Reinheit von mindestens 99,5% gewonnen, die somit intravenös anwendbar ist.

Erfindungsgemäß wird bevorzugt zunächst mit einer Anionenaustauschchromatographie begonnen, der sich eine Sterilisation, Kationenaustauschchromatographie und nachfolgend eine Reinigung an einer hydrophoben Phase anschließt.

Gegebenenfalls kann es jedoch auch von Vorteil sein, die Reinigung zunächst an einem Kationenaustauscher zu beginnen, anschließend die Sterilisation, gefolgt von der Reinigung am Anionenaustauscher und hydrophober Phase, durchzuführen. Dabei werden dann die jeweiligen, nachfolgend beschriebenen Bedingungen gewählt.

Unabhängig hiervon wurde überraschenderweise gefunden, daß durch die Kombination der genannten Verfahrensschritte eine Virusinaktivierung erfolgt, wie sie bisher bei keinem bekannten Verfahren auf der Basis eines ChromatographieSchrittes erhalten werden konnte.

Erfindungsgemäß werden IgG-MAK, bevorzugt deren Subklassen IgG₁, IgG₂, IgG₃ und IgG₄, gereinigt, wobei diese bevorzugt humanen oder tierischen, insbesondere murinen oder humanmurinen Ursprungs sein können.

Als Antikörper-haltige Lösung kann dabei die beschriebene Ascitesflüssigkeit eingesetzt werden.

Vorteilhafterweise verwendet man jedoch serumfreie, zellfreie oder definierte Medien aus der Kultivierung von Hybridomzellen oder von Epstein-Barr-Virus-Zellen (EBV-Zellen).

Die daraus gewonnenen MAK-haltigen Lösungen enthalten als Verunreinigung noch Zellproteine und DNA der Hybridome sowie den Medien zugesetzte Wachstumsfaktoren, wie z.B. humanes Transferrin und lipidkomplexierende Proteine, wie z.B. Albumin. Die Lösungen können ferner mit Viren kontaminiert sein und enthalten noch die für ein Zellwachstum notwendigen Substanzen wie Salze, Aminosäuren, Vitamine und Lipide.

Erfindungsgemäß wird die Ausgangslösung drei Chromatographie-Schritten unterworfen, nachdem die niedermolekularen Substanzen durch Diafiltration an einer Membran mit einer Ausschlußgrenze von 5-50 000 Dalton entfernt wurden, wobei gegen das 5- bis 10-fache Volumen ausgetauscht wurde. Im Anschluß erfolgt vorzugsweise zuerst eine Anionenaustauschchromatographie. Die eingesetzte Pufferlösung - vorzugsweise 5 bis 50 mmol/l Trishydroxymethylaminomethan, 0 bis 100 mmol/l Kochsalz, pH 6-8 - ist so gewählt, daß der monoklonale Antikörper an den Anionenaustauscher bindet.

Als Anionenaustauscher können kommerziell erhältliche Chromatographie-Phasen, insbesondere Q-Sephrose, QMA-Accell, DEAE-Trisacryl, DEAE-Spherosil, Q-Spherodex und DEAE-Fraktogel eingesetzt werden. Bevorzugt wird Q-Sepharose und insbesondere QMA-Accell eingesetzt.

Die mit dem monoklonalen IgG-Antikörper beladene Anionenaustauschersäule wird nachfolgend so lange mit dem Puffer gespült, bis das UV-Signal den Ausgangswert wieder erreicht hat. Dabei werden alle basischen Proteine abgetrennt. Die Elution des monoklonalen IgG-Antikörpers erfolgt anschliessend mit einem geeigneten Puffer, insbesondere der Zusammensetzung 5-50 mmol/l Trishydroxymethylaminomethan, 100-300 mmol/l Kochsalz, bei pH 6,0 bis 8,0.

Die vorgereinigte monoklonale IgG-Fraktion wird anschliessend mittels Diafiltration oder Gelfiltration auf eine Salzkonzentration von 5 bis 50 mmol/l Natriumacetat, 0-100 mmol Natriumchlorid und einen pH-Wert von 6,0 bis 8,0 umgepuffert. Die Proteinkonzentration beträgt 0,5 bis 2%. Dabei werden hierfür wiederum übliche Membranen oder Gele verwendet.

Da das Ausgangsmaterial je nach Herkunft der Hybridomlinie mit humanpathogenen Viren kontaminiert sein kann, erfolgt nach diesem ersten Chromatographieschritt nunmehr eine spezifische Sterilisation.

Hierzu können Behandlungen, insbesondere mit β-Propiolacton, TNBP (Tri-n-butylphosphat)/Tween, TNBP/Natriumcholat, TNBP, gegebenenfalls in Kombination mit UV-Bestrahlung angewendet werden.

Dabei wird β-Propiolacton bevorzugt in einer Menge von 0,02 bis 0,1% und TNBP in Mengen von 0,1 bis 1%, gegebenenfalls in Kombination mit 0,1 bis 1% Natriumcholat, eingesetzt und die Lösung bei Raumtemperatur etwa 8 bis 20 Stunden lang, wahlweise mit UV-Bestrahlung unter üblichen Bedingungen, behandelt.

Insbesondere bevorzugt erfolgt die Virusinaktivierung mit 0,1 bis 1% TNBP und 0,5 bis 2% Tween 80 bei 5°C bis 40°C, insbesondere bei 20°C bis 30°C, über 8 bis 20 Stunden. Danach wird die Lösung auf 0,05 bis 1% Protein verdünnt und der pH-Wert auf 4,5 bis 7 eingestellt. Unter diesen Bedingungen bindet der monoklonale IgG-Antikörper im folgenden Chromatographie-Schritt an einen Kationenaustauscher.

Je nach Ausgangsmaterial kann es von Vorteil sein, den Kationenaustauschschritt zuerst - also vor der Sterilisation - durchzuführen, wobei dann die hierfür angegebenen Bedingungen gewählt werden und anschließend die virusinaktivierte Lösung so eingestellt wird, daß der MAK an den dann eingesetzten Anionenaustauscher bindet.

Als Kationenaustauscher finden kommerziell erhältliche Chromatographie-Phasen, insbesondere S-Sepharose, CM-Accell, CM-Trisacryl, SP-Trisacryl, SP-Spherodex und SP-Fraktogel, vorzugsweise S-Sepharose, und insbesondere CM-Accell Verwendung.

In diesem Schritt werden alle sauren Proteine und das bevorzugt als Virusinaktivierungsmittel eingesetzte TNBP/Tween entfernt.

Den gebundenen Antikörper eluiert man mit einem geeigneten Puffer, der vorzugsweise 10 bis 40 mmol/l Natriumacetat und 50 bis 200 mmol/l Natriumchlorid bei pH 4,5 bis 9,0 enthält.

Wird in diesem Verfahrensschritt ein Anionenaustauscher eingesetzt, so wird das Sterilisationsagens dort entfernt.

Der unter diesen Bedingungen eluierte monoklonale Antikörper wird mittels Diafiltration oder Gelfiltration auf eine Salzkonzentration von 50 bis 200 mmol/l Dikaliumhydrogenphosphat und einen pH-Wert von 5,0 bis 8,0 umgepuffert.

Anschließend erfolgt eine Chromatographie an einer hydrophoben Phase. Vor diesem letzten Reinigungsschritt im erfindungsgemäßen Verfahren stellt man die Salzkonzentration der Lösung je nach verwendeter Chromatographie-Phase auf einen Wert von 1,1 bis 1,5 M/l mit festem Ammoniumsulfat ein.

Die Elution des an die hydrophobe Phase gebundenen monoklonalen IgG-Antikörpers erfolgt nach intensiver Spülung der Säule mit dem geeigneten Binde-Puffer durch einen Elutionspuffer, dessen Ammoniumsulfat-Konzentration bei 0,7 bis 1,1 M/l liegt, bei einem pH-Wert von 5 bis 8.

Als hydrophobe Chromatographie-Phase können kommerziell erhältliche Materialien wie Polypropyl-A, Polymethyl-A, Polyethyl-A, Alkyl-Sepharose und Butyl-Sepharose verwendet werden. Vorzugsweise findet Polypropyl-A Verwendung. Hierbei wird die Phase auf 1,3-1,4 M/l mit Ammoniumsulfat und der pH-Wert auf 7 eingestellt. Als Elutionspuffer eignet sich 0,1 molare K₂HPO₄-Lösung mit 1,0 M/l (NH₄)₂SO₄.

Mit diesem letzten Reinigungsschritt gelingt es, das im Kulturmedium enthaltene Transferrin und andere Proteinkontaminationen mit einem ähnlichen isoelektrischen Punkt wie der monoklonale Antikörper vollständig abzutrennen.

Die so erhaltene hochreine Antikörper-Fraktion wird mittels bekannter Methoden auf physiologisch verträgliche Bedingungen umgepuffert, sterilfiltriert und abgefüllt.

Alle Chromatographie-Schritte des erfindungsgemäßen Verfahrens werden nach dem gleichen Schema durchgeführt. Zunächst erfolgt die Bindung des Antikörpers, dann ein intensiver Waschschritt und anschließend eine Elution mit einem Stufengradienten. Dieses Verfahren hat gegenüber einer üblicherweise angewandten Elution mit einem linearen Gradienten den Vorteil, daß die Kapazität der jeweils eingesetzten Chromatographie-Phase voll ausgenutzt werden kann. Dadurch wird es möglich, große Mengen von monoklonalen IgG-Antikörpern in kurzer Zeit wirtschaftlich zu reinigen.

Je nach verwendeter Chromatographie-Phase können die chromatographischen Schritte als Normaldruck- oder Hochleistungsflüssigkeitschromatographie ausgeführt werden. Aus Gründen der Effektivität und Geschwindigkeit wird die HPLC-Methode bevorzugt.

Die Wirksamkeit des erfindungsgemäßen Verfahrens bezüglich Reinheit, DNA-Gehalt, Virusreduktion und biologischer Funktion wird anhand der folgenden Untersuchungen nachgewiesen:

Mit analytischen chromatographischen Methoden (Ausschlußchromatographie, Affinitätschromatographie und Ionenaustauschchromatographie) können keinerlei Verunreinigungen bei einem Produkt, hergestellt gemäß Beispiel 1, nachgewiesen werden.

In der SDS-Elektrophorese finden sich zwei Banden, die der leichten und schweren Kette des IgG-Moleküls zuzuordnen sind. In der isoelektrischen Fokussierung sind mehrere Banden zu erkennen, die, wie durch Western Blotting gezeigt, auf die Mikroheterogenität des Antikörpers zurückzuführen sind.

Somit führt das Verfahren unter Berücksichtigung der Nachweisgrenzen der unterschiedlichen analytischen Methoden zu einem Produkt mit einer Reinheit von mindestens 99,5%.

Die Effektivität des Reinigungsverfahrens bezüglich DNA-Entfernung wurde mit Hilfe radioaktiv-markierter DNA überprüft.

Der Reduktionsfaktor für jeden einzelnen Chromatographieschritt ergibt sich aus dem Quotienten von aufgegebener Menge markierter DNA zum DNA-Gehalt in der Antikörperfraktion.

Die Gesamtabreicherung erhält man durch Addition der Logarithmen der Einzelschritte.

Die Ergebnisse dieser Überprüfung sind in Tabelle I zusammengefaßt:

**Tabelle I**

| Chromatographiephase | Reduktionsfaktor |
|---|---|
| Anionenaustauscher gemäß Beispiel 1 | 2,5 x 10² |
| Kationenaustauscher gemäß Beispiel 1 | 6 x 10⁰ |
| Hydrophobe Phase gemäß Beispiel 1 | 2,58 x 10² |
| Gesamtreduktion | 3,5 x 10⁵ |

Wie hieraus ersichtlich ist, sind für die effektive Reduktion des DNA-Gehaltes mindestens zwei unterschiedliche Chromatographie-Schritte notwendig. Mit dem erfindungsgemäßen Verfahren wird jedoch ein DNA-Gehalt im Endprodukt erzielt, der unter den geforderten 10 pg/Dosis liegt, was der Gesamtreduktionsfaktor deutlich zeigt.

Hinsichtlich der Virusabreicherung im erfindungsgemäßen Verfahren wurden Experimente mit Modellviren für jeden einzelnen Verfahrensschritt durchgeführt.

Zur Untersuchung wurden vier verschiedene Testviren eingesetzt, die als Modell für Lipid-, Protein-, DNA- und RNA-Viren dienen.
Dabei wurde ein muriner IgG-monoklonaler Antikörper nach dem erfindungsgemäßen Verfahren wie in Beispiel 1 gereinigt.

Ein Verfahren zur Herstellung eines monoklonalen IgG-Antikörpers zur therapeutischen Anwendung muß für alle 4 Viren jeweils zu einer Reduktionsrate von mindestens 12 log-Stufen führen.

Die Ergebnisse der geschilderten Versuche sind in Tabelle II dargestellt:

**Tabelle II**

| Schritt | Parainfluenza Typ 3 | Reovirus Typ 3 | Simian-Virus SV 40 | Maus-Leukämie Virus(MuLV-Mov-3) |
|---|---|---|---|---|
| Anionenaustauscher | > 7,9 x 10⁴ | 3,7 | 1,9 x 10² | 2,6 x 10⁵ |
| Sterilisation (TNBP/Tween) | > 9,0 x 10³ | 1,2 x 10¹ | 3,5 x 10² | >5,7 x 10⁶ |
| Kationenaustauscher | > 7,1 x 10⁴ | 3,7 x 10⁵ | 4,8 x 10⁴ | 2,3 x 10⁵ |
| Hydrophobe Chromatographiephase | > 8,7 x 10³ | 4,3 x 10⁵ | 8,2 x 10² | 5,6 x 10⁴ |
| Gesamtreduktion | > 4,4 x 10¹⁷ | 7,1 x 10¹² | 2,6 x 10¹² | >1,9 x 10²² |

Man erkennt aus Tabelle II deutlich, daß die geforderte Reduktionsrate mit dem erfindungsgemäßen Verfahren erreicht wird, d.h. daß drei Chromatographieschritte dafür notwendig sind, da mit einem einzigen Schritt, wie bisher in der Literatur beschrieben, die geforderte Abreicherung nicht erfolgt.

Die Kombination von Verfahrensschritten gemäß vorliegender Erfindung führt somit zu einem monoklonalen IgG-Produkt, das allen Anforderungen für eine intravenöse Applikation gerecht wird und dessen biologische Aktivität voll erhalten ist.

Das Produkt weist eine Reinheit von mindestens 99,5% auf und kann intravenös in der Immuntherapie, z.B. bei Abstoßungsreaktionen bei der Transplantation, bei der Rhesus-Prophylaxe oder viralen Erkrankungen (z.B. gegen CMV, Hepatitis) eingesetzt werden.

Ferner kann das Produkt auch in der Immundiagnostik in üblicher Weise eingesetzt werden.

Die im Verfahren eingesetzten Chromatographie-Materialien sind billig, leicht verfügbar und können optimal ausgelastet werden. Dadurch ist es möglich, kostengünstig und wirtschaftlich hochreine IgG-MAK's in großen Mengen herzustellen, wobei überraschenderweise eine weitestgehende Virusinaktivierung erfolgt.

Die Erfindung wird durch nachstehende Beispiele erläutert.

### Beispiel 1

Eine murine Hybridomzell-Linie, die einen monoklonalen IgG₁-Antikörper gegen den humanen Interleukin-2-Rezeptor produziert, wurde in serumarmem Medium kultiviert. 518 l des zellfreien Kulturüberstandes wurden gegen 0,022 M/l Trishydroxymethylaminomethan (Tris), pH 7,5, diafiltriert und auf ein Volumen von 31 l ankonzentriert. Der IgG-Gehalt in dieser Lösung betrug 1,75 mg/ml.

Der erste chromatographische Schritt erfolgt an einer Säule, die mit 5 l Q-Sepharose fast flow gefüllt und mit 0,022 M/l Tris, pH 7,5, äquilibriert ist.

Die umgepufferte Lösung wird auf die Säule aufgegeben, wobei unter diesen Bedingungen der Antikörper an den Anionenaustauscher gebunden wird. Man spült nun mit 50 l des gleichen Puffers nach und eluiert anschließend mit 0,022 M/l Tris, 0,15 M/l NaCl, pH 7,5, den Antikörper.

Die restlichen Proteine eluieren mit 0,022 M/l Tris, 0,6 M/l NaCl, pH 7,5.

Die gesammelte Fraktion, die den monoklonalen Antikörper enthält, wird gegen 0,020 M/l Natriumacetat, pH 7,0, diafiltriert, auf eine Proteinkonzentration von 10 mg/ml ankonzentriert und über ein 0,2 µ Filter sterilfiltriert.

4,46 l der so behandelten Lösung werden bei 25°C mit TNBP auf eine Konzentration von 0,3% und mit Tween 80 auf 1% eingestellt und 16 h gerührt.

Mit 17,5 l 0,022 M/l Natriumacetat-Lösung, pH 7,0, wird auf eine IgG-Konzentration von 2 mg/ml verdünnt und der pH-Wert mit 0,1 M Essigsäure auf pH 5,5 eingestellt.

Diese Lösung wird an einer Säule, gefüllt mit 5 l S-Sepharose fast flow and äquilibriert mit 0,022 M/l Natriumacetat, pH 5,5, chromatographiert, wobei unter diesen Bedingungen der Antikörper an die Chromatographie-Phase gebunden wird. Zur Entfernung der Sterilisationsmittel und sauren Proteine wird mit 50 l Puffer nachgespült. Die Antikörper-haltige Fraktion erhält man durch Elution mit einem Puffer, der 0,022 M/l Natriumacetat, 0,075 M/l NaCl, pH 8,5, enthält.

Nach Korrektur des pH-Wertes auf 7,0 wird erneut diafiltriert mit 0,1 M/l K₂HPO₄, pH 7,0, als Substitutionslösung und sterilfiltriert.

Vor der Durchführung des dritten Chromatographieschrittes an Polypropyl-A fügt man soviel festes (NH₄)₂SO₄ zu, bis eine Endkonzentration von 1,3 M/l erreicht ist. Es folgt eine pH-Korrektur auf 7,0 und eine Sterilfiltration.

An einer HPLC-Säule, gefüllt mit 400 ml Polypropyl-A und äquilibriert mit 0,1 M/l K₂HPO₄, 1,3 M/l (NH₄)₂SO₄, pH 7,0, wird die Lösung in Portionen zu 3,4 l chromatographiert.

Zur Abtrennung von verunreinigenden Proteinen, speziell von Transferrin, spült man mit 4 l Puffer nach, bis das UV-Signal Basislinie erreicht hat. Die Elution erfolgt mit einem Puffer, der aus 0,1 M/l K₂HPO₄/0,1 M/l (NH₄)₂SO₄, pH 7,0, besteht.

Die gesammelten Antikörper-Fraktionen werden gegen 0,45% NaCl diafiltriert, mit festem Glycin auf 0,15 M/l Glycin eingestellt, die Antikörperkonzentration auf 1,1 mg/ml eingestellt und sterilfiltriert.

Die Lösung des monoklonalen IgG-Antikörpers hat folgende Eigenschaften:

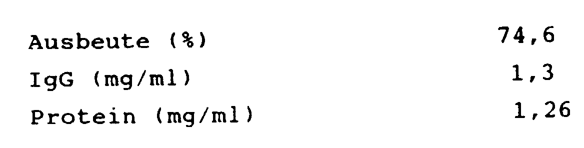

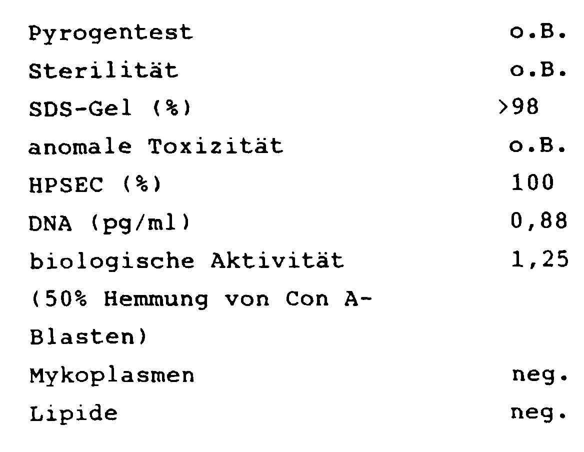

### Beispiel 2

1,64 l eines zellfreien Kulturüberstandes, der einen monoklonalen IgG-1-Antikörper gegen humanes Interleukin-6, produziert von einer murinen Hybridomlinie, enthält, wurden gegen 0,020 M/l Trishydroxymethylaminomethan (Tris) und 0,05 M/l NaCl, pH 7,0, diafiltriert und auf 0,93 l ankonzentriert. Der IgG-Gehalt in dieser Lösung betrug 2,35 mg/ml.

Der erste chromatographische Schritt erfolgt an einer Stahlsäule, die mit 50 ml QMA-Accell Plus gefüllt und mit 0,020 M/l Tris, 0,050 M/l NaCl, pH 7,0, äquilibriert ist.

Die umgepufferte Lösung wird auf die Säule aufgegeben, wobei unter diesen Bedingungen der Antikörper an den Anionenaustauscher gebunden wird. Man spült nun mit 500 ml des gleichen Puffers nach und eluiert anschließend mit 0,020 M/l Tris, 0,25 M/l NaCl, pH 7,0, den Antikörper.

Die restlichen Proteine eluieren mit 0,020 M/l Tris, 0,6 M/l NaCl, pH 7,0.

Die gesammelte Fraktion, die den monoklonalen Antikörper enthält, wird gegen 0,020 M/l Natriumacetat, 0,050 M/l NaCl, pH 7,0, diafiltriert, auf eine Proteinkonzentration von 10 mg/ml ankonzentriert und über ein 0,2 µ-Filter sterilfiltriert.

195 ml der so behandelten Lösung werden bei 25°C mit TNBP auf eine Konzentration von 0,3% und mit Tween 80 auf 1% eingestellt und 16 h gerührt.

Mit 780 ml 0,020 M/l Natriumacetat, 0,050 M/l NaCl, pH 7,0, wird auf eine IgG-Konzentration von 2 mg/ml verdünnt und der pH-Wert mit 0,1 M Essigsäure auf pH 5,0 eingestellt.

Diese Lösung wird an einer Stahlsäule, gefüllt mit 50 ml CM-Accell Plus und äquilibriert mit 0,020 M/l Natriumacetat, 0,050 M/l NaCl, pH 5,0, chromatographiert, wobei unter diesen Bedingungen der Antikörper an die Chromatographie-Phase gebunden wird. Zur Entfernung der Sterilisationsmittel und sauren Proteine wird mit 500 ml Puffer nachgespült. Die Antikörper-haltige Fraktion erhält man durch Elution mit einem Puffer, der 0,020 M/l Natriumacetat, 0,150 M/l NaCl, pH 8,0, enthält.

Nach Korrektur des pH-Wertes auf 7,0 wird erneut diafiltriert mit 0,1 M/l K₂HPO₄, pH 7,0, als Substitutionslösung und sterilfiltriert.

Vor der Durchführung des dritten Chromatographieschrittes an Polypropyl-A fügt man soviel festes (NH₄)₂SO₄ zu, bis eine Endkonzentration von 1,4 M/l erreicht ist. Es folgt eine pH-Korrektur auf 7,0 und eine Sterilfiltration.

An einer HPLC-Säule, gefüllt mit 50 ml Polypropyl-A und äquilibriert mit 0,1 M/l K₂HPO₄, 1,4 M/l (NH₄)₂SO₄, pH 7,0, wird die Lösung in Portionen zu 480 ml chromatographiert.

Zur Abtrennung von verunreinigenden Proteinen, speziell von Transferrin, spült man mit 400 ml Puffer nach, bis das UV-Signal Basislinie erreicht hat. Die Elution erfolgt mit einem Puffer, der aus 0,1 M/l K₂HPO₄/1,0 M/l (NH₄)₂SO₄, pH 7,0, besteht.

Die gesammelten Antikörper-Fraktionen werden gegen 0,45% NaCl diafiltriert, mit festem Glycin auf 0,15 M/l Glycin eingestellt, die Antikörperkonzentration auf 1,1 mg/ml eingestellt und sterilfiltriert.

Die Lösung des monoklonalen IgG-Antikörpers hat folgende Eigenschaften:

| | |
|---|---|
| Ausbeute (%) | 68 |
| IgG (mg/ml) | 1,2 |
| Protein (mg/ml) | 1,22 |
| Pyrogentest | o.B. |
| Sterilität | o.B. |
| SDS-Gel (%) | > 98 |
| anomale Toxizität | o.B. |
| HPSEC (%) | 100 |
| Lipide | neg. |

## Patentansprüche

1. Verfahren zur Reinigung von IgG-monoklonalen Antikörpern, dadurch gekennzeichnet, daß man eine diafiltrierte antikörperhaltige Ausgangslösung einer Chromatographie an einem Anionenaustauscher, einem Kationenaustauscher und einer hydrophoben Phase unterwirft, wobei die monoklonalen Antikörper an die jeweilige Chromatographie-Phase gebunden werden, selbige anschließend gewaschen und mit einem Stufengradienten eluiert werden, und die monoklonalen Antikörper zusätzlich einem spezifischen Sterilisationsschritt unterzieht.

2. Verfahren gemäß Anspruch 1, dadurch gekennzeichnet, daß man die Ausgangslösung zuerst an einem Anionenaustauscher chromatographiert, dann einer Sterilisation unterzieht, nachfolgend an einem Kationenaustauscher und anschließend an einer hydrophoben Phase chromatographiert.

3. Verfahren gemäß Anspruch 1, dadurch gekennzeichnet, daß man die Ausgangslösung zuerst an einem Kationenaustauscher chromatographiert, dann einer Sterilisation unterzieht, nachfolgend an einem Anionenaustauscher und anschließend an einer hydrophoben Phase chromatographiert.

4. Verfahren gemäß einem der Ansprüche 1 bis 3, dadurch gekennzeichnet, daß man als Ausgang eine IgG₁, IgG₂, IgG₃ oder IgG₄-haltige Lösung verwendet, wobei das IgG humanen oder tierischen Ursprungs sein kann.

5. Verfahren gemäß einem der Ansprüche 1 bis 4, dadurch gekennzeichnet, daß man als Ausgangslösung zellfreie Kulturüberstände aus der Kultivierung von Hybridomzellen oder von EBV-Zellen verwendet.

6. Verfahren gemäß einem der Ansprüche 1 bis 5, dadurch gekennzeichnet, daß man als Anionenaustauscher Q-Sepharose, QMA-Accell, DEAE-Trisacryl, DEAE-Spherosil, Q-Spherodex oder DEAE-Fraktogel, als Kationenaustauscher S-Sepharose, CM-Accell, CM-Trisacryl, SP-Triacryl, SP-Spherodex oder SP-Fractogel und als hydrophobe Phase Polypropyl-A, Polymethyl-A, Polyethyl-A, Alkyl-Sepharose oder Butyl-Sepharose verwendet.

7. Verfahren gemäß Anspruch 6, dadurch gekennzeichnet, daß man QMA-Accell, CM-Accell und Polypropyl-A als die jeweiligen Chromatographiematerialien verwendet.

8. Verfahren gemäß einem der Ansprüche 1 bis 7, dadurch gekennzeichnet, daß man den monoklonalen Antikörper zunächst an die jeweilige Chromatographie-Phase bindet, selbige dann wäscht und nachfolgend mit einem Stufengradienten eluiert.

9. Verfahren nach Anspruch 8, dadurch gekennzeichnet, daß man die Chromatographie am Anionenaustauscher bei pH 6 bis 8, am Kationenaustauscher bei pH 4,5 bis 7 und an der hydrophoben Phase bei pH 5 bis 8 beginnt und bei pH 6 bis 8, 4,5 bis 9 bzw. 5 bis 8 eluiert.

10. Verfahren gemäß einem der Ansprüche 1 bis 9, dadurch gekennzeichnet, daß man die chromatographischen Schritte jeweils als Hochleistungsflüssigkeitschromatographie durchführt.

11. Verfahren nach einem der Ansprüche 1 bis 10, dadurch gekennzeichnet, daß die Sterilisation mittels Tri-n-butylphosphat, Tri-n-butylphosphat/Tween, Tri-n-butylphosphat/Natriumcholat oder β-Propiolacton, gegebenenfalls in Kombination mit UV-Bestrahlung, erfolgt.

12. Verfahren gemäß Anspruch 11, dadurch gekennzeichnet, daß die Sterilisation mit 0,01 bis 1% Tri-n-butylphosphat/0,5 bis 2% Tween 80 bei 5°C bis 40°C, vorzugsweise 20°C bis 30°C, während 8 bis 20 Stunden erfolgt.

13. Verfahren zur Herstellung eines Mittels für die intravenöse Anwendung in der Immuntherapie oder für die Immundiagnostik, enthaltend einen IgG-monoklonalen Antikörper, dadurch gekennzeichnet, daß der IgG-monoklonale Antikörper nach dem Verfahren gemäß einem der Ansprüche 1 bis 12 hergestellt wird.

## Claims

1. Process for purifying IgG monoclonal antibodies, characterised in that a diafiltered starting solution containing antibodies is subjected to chromatography on an anion exchanger, a cation exchanger and a hydrophobic phase, wherein the monoclonal antibodies are bound to the particular chromatographic phase, the said antibodies are washed and eluted with a step gradient and the monoclonal antibodies are additionally subjected to a specific sterilisation stage.

2. Process according to claim 1, characterised in that the starting solution is first chromatographed on an anion exchanger, is then sterilised, subsequently chromatographed on a cation exchanger and then on a hydrophobic phase.

3. Process according to claim 1, characterised in that the starting solution is first chromatographed on a cation exchanger, is then sterilised, subsequently chromatographed on an anion exchanger and then on a hydrophobic phase.

4. Process according to one of claims 1 to 3,
characterised in that a solution containing IgG₁, IgG₂, IgG₃ or IgG₄ is used as the starting material, wherein the IgG may be of human or animal origin.

5. Process according to one of claims 1 to 4,
characterised in that cell-free culture supernatants from culturing hybridoma cells or EBV cells are used as the starting solution.

6. Process according to one of claims 1 to 5,
characterised in that the anion exchanger used is Q-Sepharose, QMA-Accell, DEAE-Trisacryl, DEAE-Spherosil, Q-Spherodex or DEAE-Fractogel, the cation exchanger used is S-Sepharose, CM-Accell, CM-Trisacryl, SP-Triacryl, SP-Spherodex or SP-Fractogel and the hydrophobic phase used is Polypropyl-A, Polymethyl-A, Polyethyl-A, alkyl Sepharose or butyl Sepharose.

7. Process according to claim 6, characterised in that QMA-Accell, CM-Accell and Polypropyl-A are the particular chromatographic materials used.

8. Process according to one of claims 1 to 7, characterised in that the monoclonal antibodies are first bound to the particular chromatographic phase, the said antibodies are washed and subsequently eluted with a step gradient.

9. Process according to claim 8, characterised in that chromatography is begun on the anion exchanger at pH 6 to 8, on the cation exchanger at pH 4.5 to 7 and on the hydrophobic phase at pH 5 to 8 and elution is performed at pH 6 to 8, 4.5 to 9 and 5 to 8 respectively.

10. Process according to one of claims 1 to 9, characterised in that the chromatographic stages are each performed as high performance liquid chromatography.

11. Process according to one of claims 1 to 10, characterised in that sterilisation proceeds by means of tri-n-butyl phosphate, tri-n-butyl phosphate/Tween, tri-n-butyl phosphate/sodium cholate or β-propiolactone, optionally combined with UV irradiation.

12. Process according to claim 11, characterised in that sterilisation proceeds with 0.01 to 1% tri-n-butyl phosphate/0.5 to 2% Tween 80 at 5°C to 40°C, preferably 20°C to 30°C, for 8 to 20 hours.

13. Process for the production of an agent for intravenous use in immunotherapy or for immunodiagnostics containing an IgG monoclonal antibody, characterised in that the IgG monoclonal antibody is produced using the process according to one of claims 1 to 12.

## Revendications

1. Procédé de purification d'anticorps monoclonaux de type IgG, caractérisé en ce qu'on soumet une solution initiale contenant des anticorps et soumise à une diafiltration à une chromatographie sur un échangeur d'anions, sur un échangeur de cations et sur une phase hydrophobe, les anticorps monoclonaux se liant à la phase chromatographique considérée, étant ensuite lavés et élués au moyen d'un gradient échelonné, et en ce qu'on soumet en outre les anticorps monoclonaux à une étape de stérilisation spécifique.

2. Procédé selon la revendication 1, caractérisé en ce qu'on soumet d'abord la solution initiale à une chromatographie sur un échangeur d'anions, puis à une stérilisation, ensuite à une chromatographie sur un échangeur de cations et subséquemment sur une phase hydrophobe.

3. Procédé selon la revendication 1, caractérisé en ce qu'on soumet d'abord la solution initiale à une chromatographie sur un échangeur de cations, puis à unc stérilisation, ensuite à une chromatographie sur un échangeur d'anions et subséquemment sur une phase hydrophobe.

4. Procédé selon une des revendications 1 à 3, caractérisé en ce qu'on utilise comme solution initiale une solution contenant des IgG1, des IgG2, des IgG3 ou des IgG4, l'IgG pouvant être d'origine animale ou humaine.

5. Procédé selon une des revendications 1 à 4, caractérisé en ce qu'on utilise comme solution initiale des sumageants de culture acellulaires provenant de la culture de cellules d'hybridome ou de cellules du virus Epstein-Barr.

6. Procédé selon une des revendications 1 à 5, caractérisé en ce qu'on utilise Q-Sepharose, QMA-Accell, DEAE-Trisacryl, DEAE-Spherosil, Q-Spherodex ou DEAE-Fraktogel comme échangeur d'anions, S-Sepharose, CM-Accell, CM-trisacryl, SP-Triacryl, SP-Spherodex ou SP-Fractogel comme échangeur de cations et Polypropyl-A, Polymethyl-A, Polyethyl-A, Alkyl-Sepharose ou Butyl-Sepharose comme phase hydrophobe.

7. Procédé selon la revendication 6, caractérisé en ce qu'on utilise QMA-Accell, CM-Accell et Polypropyl-A comme matériels de chromatographie respectifs.

8. Procédé selon une des revendications 1 à 7, caractérisé en ce qu'on lie d'abord l'anticorps monoclonal à la phase de chromatographie considérée, qu'on le lave alors et qu'on l'élue ensuite au moyen d'un gradient échelonné.

9. Procédé selon la revendication 8, caractérisé en ce qu'on commence la chromatographie sur l'échangeur d'anions à pH 6 à 8, sur l'échangeur de cations à pH 4,5 à 7 et sur la phase hydrophobe à pH 5 à 8 et qu'on élue à pH 6 à 8, 4,5 à 9 ou 5 à 8.

10. Procédé selon une des revendications 1 à 9, caractérisé en ce qu'on procède aux étapes de chromatographie respectives sous forme de chromatographie en phase liquide haute performance.

11. Procédé selon une des revendications 1 à 10, caractérisé en ce que la stérilisation s'effectue au moyen de tri-n-butylphosphate, de tri-n-butylphosphate/Tween, de tri-n-butylphosphate/cholate de sodium ou de β-propiolactone, le cas échéant en combinaison avec une exposition au rayonnement UV.

12. Procédé selon la revendication 11, caractérisé en ce que la stérilisation s'effectue avec 0,01 à 1 % de tri-n-butylphosphate/0,5 à 2 % de Tween 80 entre 5°C et 40°C, de préférence entre 20°C et 30°C, pendant 8 à 20 h.

13. Procédé de préparation d'un agent destiné à l'administration intraveineuse dans le traitement immunologique ou pour le diagnostic immunologique, contenant un anticorps monoclonal de type IgG, caractérisé en ce que l'anticorps monoclonal de type IgG est préparé selon le procédé selon une des revendications 1 à 12.
